(19) Europäisches Patentamt
European Patent Office
Office européen des brevets

(11) EP 4 089 163 A1

(12) EUROPEAN PATENT APPLICATION

(43) Date of publication:
16.11.2022 Bulletin 2022/46

(21) Application number: 21305612.0

(22) Date of filing: 11.05.2021

(51) International Patent Classification (IPC):
*C12N 5/00* (2006.01) *A61L 27/52* (2006.01)
*C08L 5/00* (2006.01) *C08B 37/00* (2006.01)
*C08B 37/08* (2006.01) *C08L 5/08* (2006.01)
*A61K 47/36* (2006.01)

(52) Cooperative Patent Classification (CPC):
(C-Sets available)
**C08L 5/00; A61L 27/20; A61L 27/38; A61L 27/52; B33Y 70/00; C08B 37/0063; C08B 37/0072; C08L 5/08; C12N 5/0062** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Université Clermont Auvergne
63000 Clermont-Ferrand (FR)**
- **Université de Picardie Jules Verne
80025 Amiens Cedex 1 (FR)**
- **Osaka University
Suita-shi
Osaka 565-0871 (JP)**

(72) Inventors:
- **DELATTRE, Cédric
63110 BEAUMONT (FR)**
- **PIERRE, Guillaume
63730 Les Martres-de-Veyre (FR)**
- **MICHAUD, Philippe
63160 BILLOM (FR)**
- **PETIT LAIGNEL, Emmanuel
80680 HEBECOURT (FR)**
- **EL BOUTACHFAITI, Redouan
80000 AMIENS (FR)**
- **SAKAI, Shinji
OSAKA, 560-0053 (JP)**

(74) Representative: **Novagraaf Technologies
Bâtiment O2
2, rue Sarah Bernhardt
CS90017
92665 Asnières-sur-Seine Cedex (FR)**

(54) **POLYMERIC COMPOUND OF GLUCURONIC ACID WITH PHENOLIC GROUPS, GEL-FORMING COMPOSITION COMPRISING SUCH A COMPOUND AND METHOD FOR PRODUCING THE SAME**

(57) The present invention relates to polymeric compounds of glucuronic acid having phenolic hydroxyl moieties (herein after designated by PGU-Ph) and a method of hydrogelation of such compounds. The present invention also relates to the use of the hydrogel structures (particles, films or 3D-strucutres) obtained by this method, notably as three-dimensional cell culture material to support biological molecules used as active ingredients, cell colonisation or for tissue regeneration

EP 4 089 163 A1

(52) Cooperative Patent Classification (CPC): (Cont.)

C-Sets
**A61L 27/20, C08L 5/00**

## Description

### TECHNICAL FIELD

The present invention relates generally to polymeric compounds of glucuronic acid and a method of hydrogelation of such compounds. The present invention also relates to the use of the hydrogel structures (particles, films or 3D-strucutres) obtained by this method, notably as three-dimensional cell culture material to support biological molecules used as active ingredients, cell colonisation or for tissue regeneration.

### BACKGROUND

Polyglucuronic acids (PGU) also called glucuronan is a homopolymer of glucuronic acid composed of [→4)-β-D-GlcpA-(1→] residues partially acetylated at the C-3 and/or the C-2 position produced by the strain *Sinorhizobium meliloti* M5N1CS[1]. First described in cell walls of *Mucor rouxii*[2], these polyuronides have since been isolated from other sources such as in the cell walls of green algae[3]. However the most described polysaccharide was obtained by the *Rhizobia* strains. However recent progresses in the oxidation of primary hydroxyls groups by 2,2,6,6-tetramethylpiperidine-1- oxyl radical (TEMPO) reagents permits to obtained PGU mimick derivatives from cellulose, xanthan, curdlan, scleroglucan, chitosan, starch, fungal $\alpha$-(1,3)-glucan, etc. on a large scale-up and concomitantly new polysaccharide lyase family able to degrade these polyglucuronic acids have been identified[4],[5].

In the field of poly-and oligo-glucuronic acids, different applications of these compounds, and in particular the French patent FR2781673[6] and the international application WO 1993/18174[7] teach the biocompatibility of PGU and its use in food products, farming, pharmaceutics, cosmetics or water purification, particularly as a gelling, thickening, hydrating, stabilizing, chelating or flocculating agent. Another application concerned the immunostimulating properties on human blood monocytes, low molecular weight PGU enhanced the production of cytokines IL-1, IL-6 and TNF-α[8]. Cosmetics application of PGU have been claimed by Lintner (1999)[9] in association with an algae extracted from *Haematococcus pluvialis* or in WO2010/067327[10] for oligo-PGU stimulating of elasticity of the dermis and epidermis. Biological activities of these low molecular weight glucuronans modified by sulphonation were also investigated on a model of injured extensor digitorum longus (EDL) muscles on rats and demonstrated that the regeneration activity is not induced only by the presence of sulfate groups, but also by acetyl groups. The renewal process of cells is regulated by specific signals (or communication peptides such as growth factors) of the extracellular matrix. These signals are stored, protected, and positioned on a family of large polysaccharides called Heparan Sulfates (HS). In cases of injury, specific enzymes destroy HS, that no longer protect the specific signals. Other enzymes called proteases then destroy specific signals along with other structural proteins of the extracellular matrix. Due to their resistance against natural enzymes from the extracellular matrix, the biological effect of these modified bacterial polysaccharides could be explained[11].

In that context, the Applicant has recently discovered the hydrogelation property of PGU or PGU derivatives having phenolic hydroxyl moieties (herein after designated by PGU-Ph) and their potential as components of bioinks for bioprinting. Thus the Applicant has further developed a method for hydrogelating these PGU-Ph compounds in which the phenolic hydroxyl moieties allow a rapid formation of stable hydrogels through horseradish peroxidase (HRP)-catalyzed crosslinking.

HRP-assisted hydrogelation is already known by the man of the art as an effective method for obtaining cell-laden hydrogels from a variety of derivatives of natural and synthetic polymers such as alginate[12], hyaluronic acid[13], gelatin[14], dextran[15], and poly (vinyl alcohol)[16]. Recently, HRP-assisted hydrogelation was applied to 3D bioprinting[17],[18], in which rapid curation of inks ejected from needles are required for fabricating 3D constructs with higher fidelity to blueprints. 3D bioprinting is a known technique of fabrication of cell-laden constructs based on digital blueprints. The resultant cell-laden constructs are fabricated for the sake of wound dressing and tissue engineering for drug screening and regenerative medicine[19],[20].

The applicant has discovered that the addition of Ph clusters to PGU or its derivatives allows the use of a particular (enzymatic) cross-linking pathway which dispenses with using inorganic crosslinkers which are slower and blur the structure formed. The hydrogel structures thus obtained form a transparent network and with such a good retention of the printed forms.

### SUMMARY OF THE INVENTION

Consequently, the Applicant has developed a polymeric compound of glucuronic acid comprising at least one phenolic group (hereinafter designated by the acronym PGU-Ph).

According to a first embodiment of the invention, the phenolic group may be a phenolic hydroxyl group X-(Y-amino-Z-hydroxyalkyl)-n-hydroxyl-phenol of formula (I)

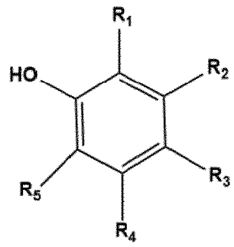

(I)

with:

- $R_1$, $R_2$, $R_4$ and $R_5$ designating a possible hydroxyl group and/or a hydrogen, and/or carboxyl and/or carbonyle and/or alkylphenol and/or aminoalkyl and/or $C_1$ to $C_{20}$ aminohydroxyalkyl and/or $C_1$ to $C_{20}$ aminoalkylphenol
- n designating the number of possible hydroxyl groups and being an integer varying from 0 to 4,
- $R_3$ designating a $C_1$ to $C_{20}$ aminohydroxyalkyl group or a $C_1$ to $C_{20}$ aminoalkyl.

Preferably, the phenolic group of the polymeric compound of glucuronic acid according to the first embodiment of the invention may be selected from the group constituted by tyramine, dopamine and octopamine, and even more preferably tyramine.

Preferably, according to the first embodiment of the invention, the phenolic hydroxyl group of formula (I) may be grafted on a α or β-D-glucuronic unit of formula (B)

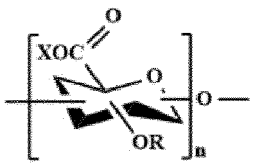

**(B)**

or its monovalent ion carboxylate salt of formula (A)

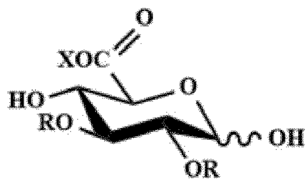

**(A)**

being bound either:

- (1) in β-(1,4), such as the oxidised forms of cellulose and its derivatives (for instance xanthan) ; and/or
- (2) in α-(1,4), such as the oxidised forms of starch and its derivatives; and/or
- (3) in β-(1,3) such as the oxidised forms of curdlan and its derivatives (for instance scleroglucan); and/or
- (4) in α-(1,3) such as the oxidised forms of fungal alpha-glucans; and/or
- (5) in α-(1,2); and/or
- (4) in β-(1,4) such as bacterial glucuronans synthesised by *Sinorhizobium meliloti* M5N1CS;

with:

X= designating monovalent ion M+ of a metal belonging to the group of alkali metals, such as such as Na+ or K+,and
R= designating H and/or sulfate and/or an acyl group including $COCH_3$, and
n being an integer chosen so that the molar mass of the polymeric compound is comprised between 10 and 1000 kilodaltons.

The Applicant has thus developed a method for producing a polymeric compound of glucuronic acid PGU-Ph according to the first embodiment of the invention (hereinafter designated by first method for producing a PGU-Ph), which comprises the following steps:

- providing a polymeric compound of glucuronic acid (herein after designated by the acronym PGU) of formula (A) or (B) as defined above;
- dissolving the PGU in an acid buffered solution for forming a solution of PGU, preferably in a 2-(N-morpholino)ethanesulfonic acid (MES) buffered solution (pH6.0, 100 mM) at 1 w/v%;
- sequentially adding, in said solution of PGU, a X-(Y-amino-Z-hydroxyalkyl)-n-hydroxyl-phenol or a salt derivative thereof (such as hydrochlorides), N-Hydroxysuccinimide (NHS) and ethyl-3-(3-dimethylaminopropyl)carbodiimide (WSCD), for forming the final composition of the reaction medium;
- stirring said final composition (preferably for at least 4 hours (and preferably 20 hours) at room temperature (i.e. between 20°C and 25°C) until obtaining a resultant polymer consisting in a polymeric compound of glucuronic acid comprising at least one phenolic hydroxyl moieties.

The last step of stirring may be followed by a step of precipitating said resultant polymer in acetone and then washing it with an 90% ethanol + 10% water until the absorbance at 275 nm attributed to the existence of tyramine became undetectable in the washing solution, as shown in example 1.

Preferably, in the method for producing a polymeric compound of glucuronic acid PGU-Ph according the first embodiment of the invention, the X-(Y-amino-Z-hydroxyalkyl)-n-hydroxyl-phenol may be selected from the group constituted by tyramine, dopamine and octopamine, and even more preferably tyramine.

The Applicant has also developed another method for producing a polymeric compound of glucuronic acid PGU-Ph according to a second embodiment of the invention (hereinafter designated by second method for producing a PGU-Ph), which comprises the following steps:

- providing a radical of a polymeric compound of glucuronic acid of formula (C)

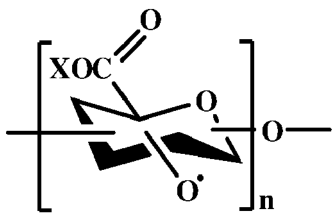

(C);

- reaction of said radical of formula (C) with a phenolic compound of formula (I) (see above) or (D)

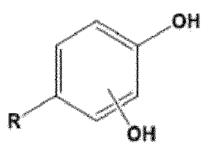

(D)

by radical coupling so as to obtain a polymeric compound of glucuronic acid of formula (E) or (F) respectively

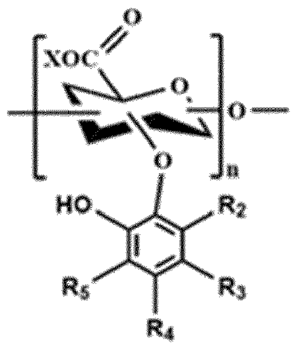

(E).

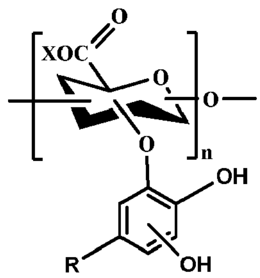

(F)

Preferably, in the method for producing a polymeric compound of glucuronic acid PGU-Ph according to the second embodiment of the invention, the phenolic compound of formula (D) may be selected from the group constituted by tyramine, dopamine and octopamine, and preferably tyramine. But it is possible to use any phenolic compound.

Another object of the invention is an aqueous solution comprising a polymeric compound of glucuronic acid of the invention (atypically in an amount of 0.5 to 8 w/v%) obtainable by any of the methods for producing such a polymeric compound according the first and second embodiments. The drying of such a solution once coated on a flat surface leads to a water soluble ungelled films of PGU-Ph (which are just dried, but not cross-linked). A cross-link process could be produce on dried film using classical cross-linker solution composed of divalent ion ($M^{2+}$ such as preferentially $Ca^{2+}$ or $Mg^{2+}$). The reticulation of PGU-Ph solutions could be performed using:

(1) a cross-link catalyst consisting in divalent cation ($M^{2+}$) such as Ca2+, Mg2+ etc., or
(2) a photo-gelification process under visible and/or UV light using photoinitiator such as riboflavin, ruthenium II trisbipyridyl chloride ([Ru(bpy)3]2+), or their derivatives.

Still another object of the invention is a gel-forming composition comprising:

- a polymeric compound of glucuronic acid according to the invention or obtainable by the methods according to the first and second embodiments of the invention, and
- a cross-linking catalyst consisting in an oxidase or a compound (in liquid or gaseous form) containing $H_2O_2$ or capable of generating $H_2O_2$ *in situ* (for instance reducing molecules such as glucose, fructose or galactose (monosaccharide), or lactose and maltose (disaccharides)).

In this gel-forming composition, if the compound containing $H_2O_2$ or capable of generating $H_2O_2$ is glucose (or fructose, or galactose) the cross-linking catalyst may further contain glucose oxidase (or fructose oxidase, or galactose oxidase respectfully), $H_2O_2$ is generated more rapidly.

According to a first embodiment of the gel-forming composition of the invention, the polymeric compound of glucuronic acid PGU-Ph may be associated to an oxidase as cross-linking catalyst, said oxidase being chosen from the group consisting of oxido-reductase, peroxidase, catalase, tyrosinase and/or monosaccharide oxidase, and the mixtures thereof.

Preferably, the oxidase may be a horseradish peroxidase (hereinafter designated by the acronym HRP) containing enzyme in an amount of at least 0.1 U/mL, preferably comprised between 0.1 U/mL and 20 U/mL in the composition for an efficient and fast cross-linking, and even better in the order of 5 U/mL. Below 0.1 U/mL, the gelling is very slow

and above 200 U/mL, the gelling is too fast and difficult to be controlled. The enzyme unit (U) is defined as follows: one pyrogallol unit will form 1.0 mg purpurogallin from pyrogallol in 20 sec at pH 6.0 at 20 C.

According to a second embodiment of the gel-forming composition of the invention, the polymeric compound of glucuronic acid PGU-Ph may be associated to a compound containing $H_2O_2$ in an amount comprised between 0.05 mmol/L and 1 mmol/L, which leads, in presence of HRP to a well-controlled gelling for a gelling time of 2s to 5 s. Outside this range, notably from 0.01-0.05 mmol/L and higher than 1 mmol/L, the gelling is much more slower (gelling time 15 s order of magnitude); but such an amount of a compound containing $H_2O_2$ could be interesting for applications requiring handling time e.g. hydrogel film making.

For either the first or the second embodiment of the gel-forming composition of the invention, the amount of polymeric compound of glucuronic acid PGU-Ph may be comprised between 0.01 w/v % to 8 w/v %, and preferably 0.1 w/v % to 2 w/v %. If the amount of polymeric compound of glucuronic acid PGU-Ph is 0.01 w/v %, the gelling is feasible but very slow. Above 8 w/v % of polymeric compound of glucuronic acid, the gel-forming composition of the invention is very viscous and difficult to use. Furthermore, it is difficult for a crosslinker to penetrate the composition.

According to a first variant of the gel-forming composition of the invention which is applicable to both embodiments, the gel-forming composition of the invention, may further comprise another active biodegradable polymers. Advantageously, the biodegradable polymers may be a polysaccharide such as glycosaminoglycan, or a protein selected from the group consisting of collagen, adhesin, gelatin, and the mixtures thereof.Preferably, the biodegradable polymer may be a gelatin derivative comprising phenolic hydroxyl moieties (Gelatin-Ph) .

According to a second variant of the gel-forming composition of the invention which is also applicable to both embodiments, the gel-forming composition of the invention, may further comprise suspended cells of animal, bacterial or plant origin. These may be 10T1/2 cells or HepG2 cells, present in said composition at a concentration in the range of $3.10^5$ cells/mL.

Another object of the invention is a method for manufacturing a hydrogel structure (hereinafter designated by first method for manufacturing a hydrogel structure), comprising the following steps:

- providing a gel-forming composition according to the first variant,
- hydrogelating said gel-forming composition:

    ◦ either by contact with a fluid or gaseous medium containing $H_2O_2$ if the gel-forming composition comprises an oxidase;
    ◦ or by contact with an oxidase if the gel-forming composition comprises a compound containing $H_2O_2$ or capable of generating $H_2O_2$ *in situ.*

Still another object of the invention is another method for manufacturing a hydrogel structure (hereinafter designated by second method for manufacturing a hydrogel structure), comprising the following steps:

- providing a gel-forming composition according to the second variant,
- hydrogelating said gel-forming composition:

    ◦ either by contact with a fluid or gaseous medium containing H2O2 if the gel-forming composition comprises an oxidase;
    ◦ or by contact with an oxidase if the gel-forming composition comprises a compound containing H2O2 or capable of generating H2O2 in situ.

Both methods may consist in a bioprinting process, for manufacturing:

- one dimensional hydrogel structures such as particles (micro- and nanoparticles): for instance by using various processes (micellar solutions, drop by drop in a cross-linking solution; or
- two-dimensional hydrogel structures such as lattices/films: for instance by using classic shaping processes (spin coating, tape casting, etc.; or
- three-dimensional hydrogel structures for instance by using three-dimensional bioprinting, preferably a process selected from the group consisting of inkjet bioprinting, extrusion bioprinting, stereolithography bioprinting, and laser-assisted bioprinting.

These examples are by no means exhaustive and are given by way of example only.

Another object of the invention is a hydrogel obtainable by the first method for manufacturing a hydrogel structure, regardless of the bioprinting process used. The t obtained hydrogel structure may be used as cell culture material to support cell colonisation (3D application) or as a patch or a plaster (2D application).

Still another object of the invention is a hydrogel structure obtainable by the second method for manufacturing a hydrogel structure, regardless of the bioprinting process used. The thus obtained hydrogel structure may be used for tissue regeneration.

## BRIEF DESCRIPTION OF THE FIGURES

Other innovative features and advantages of the invention will emerge from a reading of the following description followed by way of indication and in no way imitatively, with reference to the examples and corresponding figures. The figures are presented below:

- FIG.1 shows the UV-Vis absorbance spectra of PGU and PGU-Ph at 0.1 w/w% (see example 1);
- FIG.2 shows the shear rate-viscose profiles of PGU and PGU-HPh at 1 and 2 w/v% (see example 1);
- FIG.3 shows the effect on gelation of a) PGU-Ph at 5 U/mL HRP and 0.1 mM $H_2O_2$, and the effect of b) HRP at 1 w/v% PGU-HPh and 0.1 mM $H_2O_2$, as well as the effect of c) $H_2O_2$ at 1 w/v% PGU-HPh and 5 U/mL HRP (see example 2);
- FIG. 4 shows the morphologies of cells and mitochondrial activity of 10T1/2 cells at 20 hours of culture in the mixture solutions of medium (50 vol%) and PBS (50 vol%) containing PGU or PGU-Ph after 0.5 w/v% (see example 3);
- FIG.5 are microphotographies of a) 10T1/2 cells at 1, and 4 days, and b) HepG2 cells at 1, and 3 days of seeding on cell culture dish (Dish), PGU-Ph hydrogel, and PGU-Ph + Gelatin-Ph hydrogel (Bars: 100 pm) (see example 4);
- FIG.6 are merged microphotographies of a) 10T1/2 cells and b) HepG2 cells enclosed in PGU-Ph hydrogels through bioprinting at 1, 4 and 8 days of printing. The cells were stained using calcein-AM (Green) and PI (red) (Bars: 200 pm) (see example 5);
- FIG.7 are examples of 3D-constructions of PGU-Ph hydrogel using blue print 3D CAD-Model (see example 6);
- FIG.8 are PGU-Ph beads obtained by dropping PGU-Ph+HRP solution in $H_2O_2$ (0.5 M) (see example 7);
- FIG. 9. is antioxidant PGU-Ph film obtained by drying a solution of PGU-Ph (1 w/v %)(see example 8).

Figures 1 to 9 are described in more detail in the examples which follows, given by way of indication, and which illustrates the invention, but without limiting the scope thereof.

## EXAMPLES

### MATERIALS AND TESTS

#### Materials

- Tyramine hydrochloride purchased from Combi-Blocks (San Diego, CA);
- water-soluble carbodiimide (WSCD) purchased from Peptide Institute (Osaka, Japan);
- N-Hydroxysuccinimide (NHS), HRP (210 units/mg), and $H_2O_2$ aqueous solution (31 w/w%) purchased from Fujifilm Wako Pure Chemical Industries (Osaka, Japan);
- Mouse fibroblast 10T1/2 cells and human hepatoma HepG2 cells obtained from the Riken Cell Bank (Ibaraki Japan), which were grown in Dulbecco's modified Eagle's medium (DMEM, Nissui, Tokyo, Japan) supplemented with 10 v/v% feal bovine serum in a 5 % $CO_2$ incubator:
- *Sinorhizobium meliloti* M5N1CS (or S. *meliloti* M5N1CS strain).

#### PGU production and extraction

- The *S. meliloti* M5N1CS strain was grown at 30°C in a 20 L bioreactor (SGI) with 15 L of Rhizobium complete medium, supplemented with sucrose 1% (w/v) (RCS medium);
- The inoculum was a 1.5 L of RCS medium inoculated with *S. meliloti* M5N1CS, and was incubated during 20 hours at 30°C on a rotary shaker (120 rpm);
- After 72 h of incubation, the obtained broth was centrifuged at 33,900 x g during 40 min at 20°C. The supernatant was purified by tangential ultrafiltration on a 100,000 normal-molecular-weight cutoff (NMWCO) polyethersulfone membrane from Sartorius (Goettingen, Germany) against distilled water;
- Finally, the retentate solution was freeze-dried to obtain a PGU of formula (B) having a β-(1,4)-D-polyglucuronic

acid chainwith *O*-acetylation at carbon 2 and carbon 3 of $\beta$-D-glucuronic acid units.

## TESTS

### Shear rate-viscosity profile

Shear rate-viscosity profiles of solutions were measured using a rheometer (HAAKE MARS III, Thermo Fisher Scientific, Waltham, MA) equipped with a parallel plate of a 25-mm radius with a 0.5-mm gap at 20°C.

### Hydrogelation time

- The gelation time was measured for a phosphate-buffered saline solution (50-100 mM, pH 7.4) containing PGU-Ph at room temperature;
- This PGU-Ph solution was poured into a 24-well plate at 0.2 mL/well;
- Then, 0.1 mL of HRP and 0.1 mL $H_2O_2$ solutions were sequentially added into well and stirred using a magnetic stirrer bar (10 mm long);
- The gelation was confirmed when magnetic stirring was hindered and the surface of the solution swelled.

### Cytocompatibility

- 10T1/2 cells were seeded in the wells of 96-well cell culture plate at $4 \times 10^3$ cells/well and incubated in medium for 20 hours in a humidified 5% $CO_2$ incubator at 37°C;
- Subsequently, the medium was changed to the medium (0.2 mL) containing PGU or PGU-Ph at 0.5 w/v % and incubated for an additional 24 hours;
- Then, the medium containing the polymers were changed to the medium (0.2 mL) containing 1/20 vol of the reagent from a colorimetric mitochondrial activity assay kit (purchased under the commercial name Cell Counting Kit-8, by the Japanese firm Dojindo, Kumamoto, Japan);
- After 2 hours of incubation, the absorbance at 450 nm was measured using a spectrophotometer;
- Sodium alginate (Alg) and alginate possessing Ph moieties (Alg-Ph) were used as controls.

### Cell behavior on hydrogels

- A solution containing 1 w/v% PGU-Ph, or 1 w/v% PGU-Ph + 1 w/v% Gelatin-Ph, and 5 U/mL HRP was poured into the wells of 12-well cell culture dish at 0.5 mL/well;
- Subsequently, the dish was put in a plastic container;
- Air containing 8 ppm $H_2O_2$, obtained by bubbling air in a 0.5 M aqueous $H_2O_2$ solution, flows into a plastic container at 10 L/min ;
- After 15 min of the exposure to the air containing H2O2, the wells coated with hydrogels were rinsed sequentially with PBS and medium ;
- 10T1/2 cells and HepG2 cells were suspended in medium containing 0.3 mg/mL of catalase, and poured into each well at $6 \times 10^4$ cells/well.

## PRINTING PROCESS AND SYSTEM

- An extrusion 3D printing system that has been developed by modifying a commercial 3D printing system (purchased under the commercial name Anycubic i3 Mega by the firm Anycubic, Guangdong, China) is used for 3D bioprinting.
- This extrusion 3D printing system consisted of a syringe pump for flowing ink, a 27-gauge stainless steel needle for extruding the ink, a bubbling system for supplying air containing 8 ppm $H_2O_2$, and a stage for layering the extruded ink. The flow rates of inks in the needle and the moving speed of the stage were fixed at 22 mm/s.
- The printing of cell-laden 3D hydrogel constructs was performed in a biological safety cabinet.

- Inks containing 1 w/v% PGU-Ph, or 1 w/v% PGU-Ph and 1 w/v% Gelatin-Ph, and 5 U/mL HRP were used.
- The effect of the extrusion with the inks on cells was determined by measuring the viabilities of 10T1/2 cells and HepG2 cells suspended in the inks at 3× 105 cells/mL.
- The inks containing cells were collected at the tip of the needle and the cells were stained with trypan blue dye for the measurement using a hemocytometer.
- The viabilities of the cells enclosed in the hydrogels obtained through the printing process were determined by staining the cells with fluorescence dyes, calcein-AM and propidium iodide (PI).

**EXAMPLE 1: PGU-PH SYNTHESIS REALIZED ACCORDING TO THE FIRST METHOD FOR PRODUCING A PGU-PH**

This synthesis is realized according to the first method for producing a PGU-Ph, as follows:

- This resulting was dissolved in 2-(N-morpholino)ethanesulfonic acid (MES) buffered solution (pH6.0) at 1 w/v%.
- Tyramine hydrochloride, NHS, and WSCD were sequentially added at 45 mM, 10 mM, and 20 mM, respectively, and stirred for 20 h at room temperature;
- The resultant polymer was precipitated in acetone and then washed with 90% ethanol and 10% water until the absorbance at 275 nm attributed to the existence of tyramine became undetectable in washing solution;
- The resulting phenolized PGU is a PGU-Ph according the first embodiment of the invention.

Fig. 1 shows the UV-vis spectrum of a 0.1 w/w% PGU-Ph solution as described above (PGU-Ph solution), which is compared to that of 0.1 w/w% PGU (PGU solution). FIG.2 notably shows that the PGU solution does not have a peak around 275 nm, whereas PGU-Ph solution had a peak around 275 nm attributed to Ph moieties. The contents of Ph moieties calculated on the basis of a calibration curve obtained from a known percentage of tyramine solution is $3.7\times 10^{-4}$ mol-Ph/g.

Fig. 2 shows the shear rate-viscosity profiles of 1 and 2 w/v% PGU-Ph solutions, which are compared to the shear rate-viscosity profiles of 1 and 2 w/v% PGU solutions. Fig.2 notably shows that the viscosities of the PGU-Ph solutions are higher than those of the PGU solutions, and the viscosity of the 2 w/v% PGU-Ph solution is higher than that of the 1 w/v% PGU-Ph solution.

**EXAMPLE 2: Hydrogelation of PGU-Ph solutions**

Hydrogels were produced in accordance with the first method for manufacturing a hydrogel structure according to the invention, using the PGU-Ph solutions obtained in Example 1 by HRP-catalysed reaction in the presence of $H_2O_2$.

Fig. 3a shows the effect of PGU-Ph concentration on hydrogelation time at 5 U/mL HRP and 0.1 mM $H_2O_2$. The gelation time of a PGU-Ph solution at 0.5 w/v% is 6.0 s.

Fig. 3b and 3c show the effects of HRP and $H_2O_2$ concentrations on gelation time measured for 1.0 w/v% PGU-Ph solutions. Gelation time decreases as HRP concentration increases from 71 s at 0.1 U/mL to 2 s at 20 U/mL (Fig. 3b). Gelation time decreases as $H_2O_2$ concentration increases from 0.05 mM to 1 mM, but increases as $H_2O_2$ concentration further increases. Note to mention that higher concentration of $H_2O_2$ will lead to depolymerisation of PGU-Ph and then reduce considerably the gelification.

**EXAMPLE 3: Cytocompatibility of PGU-Ph**

For evaluating the cytocompatibility of the PGU-Ph obtained at example 1, 10T1/2 cells were incubated in a solution containing PGU-Ph. Solutions containing PGU, Alg, or Alg-Ph were used as controls.

Fig. 4a, b, c show the morphologies and mitochondrial activities of cells at 20 h of culture in the mixture solutions of medium (50 vol%) and PBS (50 vol%) containing either the PGU-Ph of example at 0.5 w/v% or tis corresponding PGU.

There are no remarkable differences in cell morphology specific to the exposure to PGU-Ph. In addition, there was no significant decrease in the mitochondrial activity of cells incubated in the mixture solutions caused by Ph moieties introduced in PGU ($p = 0.45$), as the same with the cells incubated in the mixture solutions containing 0.5 w/v% Alg and Alg- Ph ($p = 0.28$, Fig. 4c).

The mitochondrial activities of the cells incubated in the solutions containing PGU and PGU-Ph were about 20% higher than those incubated in the solutions containing Alg and Alg-Ph ($p < 0.03$).

**EXAMPLE 4: Cell behavior on PGU-Ph hydrogels**

Hydrogels containing PGU-Ph alone (as obtained in example 1) and Hydrogels containing both PGU-Ph (as obtained in example 1) and Gelatin-Ph were used for evaluating cytocompatibility and cell adhesiveness of hydrogels

containing PGU-Ph. The day after seeding, majority of 10T1/2 cells and HepG2 cells were floating on PGU-Ph hydrogels, and HepG2 cells formed aggregates (Fig. 5a, 5b) .

During the subsequent incubation period, the cells continued to float on PGU-Ph hydrogels. A small number of cells adhered to the hydrogels, but did not elongated. In contrast, the 10T1/2 cells seeded on PUG-Ph + Gelatin-Ph hydrogels adhered, elongated and proliferated as the same with those on cell culture dish (Fig. 5a).

No remarkable morphological difference was found between the 10T1/2 cells on the PUG-Ph + Gelatin-Ph hydrogels and cell culture dish. The HepG2 cells seeded on PGU-Ph + Gelatin-Ph hydrogels also adhered, elongated and proliferated (Fig. 5b). However, their morphologies were obviously different from those on cell culture dish. The HepG2 cells on cell culture dish formed small aggregates with adhering to the substrate the day after seeding. Then, the cells grew as monolayers with increasing the size of aggregates. The HepG2 cells on PGU-Ph + Gelatin-Ph hydrogels did not form obvious aggregates the day after seeding.

In the subsequent incubation period, the HepG2 cells grew on the PGU-Ph + Gelatin-Ph hydrogels without forming obvious aggregates, far from the aggregates formed on cell culture dish.

**EXAMPLE 5: 3D printing of hydrogel as cell culture materials to support cell colonisation.**

The effects of the 3D printing process and the PGU-Ph hydrogels on cells were evaluated by printing hydrogel constructs enclosing 10T1/2 cells and HepG2 cells. The viabilities of 10T1/2 cells and HepG2 cells the day after bioprinting determined through the staining with calcein-AM and PI were 92.3% and 91.6%, respectively. This result demonstrate the printing process using PGU-Ph solution as ink was not harmful for these cells.

Regarding the morphologies of the enclosed cells, 10T1/2 cells kept round shape during 8 days of study without a formation of cell aggregates (Fig. 6a). In contrast, HepG2 cells formed aggregates in the hydrogel constructs and the size of the aggregates increased with increasing culture period (Fig. 6b). There was no obvious increase in dead cells in both the cells.

**EXAMPLE 6: 3D printing of hydrogel.**

For evaluating the feasibility of PGU-Ph solution as inks of bioprinting, 1 w/v% PGU-Ph solution containing 5 U/mL HRP was extruded on substrates. As shown in Fig. 7, transparent 3D-hydrogel constructs with good fidelity to blue prints (3D CAD models) were obtained when the solution was extruded in air containing 8 ppm $H_2O_2$. These results demonstrate the feasibility of PGU-Ph solution gellable through HRP-mediated hydrogelation as inks of 3D printing.

**EXAMPLE 7: Synthesis of particles.**

For evaluating the feasibility of PGU-Ph particles synthesis such as microbeads, 1 w/v% PGU-Ph solution containing 5 U/mL HRP was dropped in $H_2O_2$ (0.5 M) and stirred for 1 minute. Then, the microbeads were washing with water, and was preserved in ethanol/water solution (70/30). As shown in Fig. 8, transparent PGU-Ph beads with good spheric shapes were obtained.

**EXAMPLE 8: Synthesis of antioxidant PGU-Ph dried films.**

For evaluating the feasibility of antioxidant PGU-Ph film synthesis, 1 w/v% PGU-Ph solution was pouring in plastic Petri dishes and dried at 50 °C during 24 h. PGU film without Ph moieties was used as control. To estimate the antioxidant effect of PGU-Ph film, the free radical scavenging activity was measured using 1,1-diphenyl-2-picrylhydrazyl (DPPH). Briefly, PGU-Ph film (100 mg) were added into 5.0 ml of DPPH solution (0.1 mM DPPH in ethanol 96°). The solution was left for 24 h under stirring at room temperature in the dark. Then, the absorbance was measured at 517 nm using the Shimadzu UV-1700 spectrophotometer.

DPPH radical scavenging activity was calculated as an inhibition percentage based on the following equation (1):

$$\text{DPPH radical inhibition } (\%) = ((A_{control} - A_{sample})/A_{control}) \times 100 \quad (1)$$

(1)

Where $A_{sample}$ and $A_{control}$ are the absorbance at 517 nm for the PGU-Ph film and for the control without PGU-Ph film (i.e.,the DPPH solution) respectively.

As shown in Fig. 9a, transparent PGU-Ph film was obtained. As observed in Fig. 9b, these results demonstrate the feasibility of PGU-Ph solution as antioxidant film for food packaging and biomaterial.

**LIST OF REFERENCES**

1- Heyraud, A., Courtois, J., Dantas, L., Colin-Morel, P., & Courtois, B. (1993). "Structural characterization and rheological properties of an extracellular glucuronan produced by a Rhizobium meliloti M5N1 mutant strain". Carbohydrate Research, 240(24), 71-78.

2- De Ruiter, G.A., Josso, S.L., Colquhoun, I.J., Voragen, A.G.J., & Rombouts, F.M. (1992). "Isolation and characterization of β-(1-4)-D-glucuronans from extracellular polysaccharides of moulds belonging to Mucorales". Carbohydrate Polymers, 18(1), 1-7.

3- Elboutachfaiti, R., Delattre, C., Petit, E., El Gadda, M., Courtois, B., Michaud, P., El Modafar, C., & Courtois, J. (2009). "Improved isolation of glucuronan from algae and the production of glucuronic acid oligosaccharides using a glucuronan lyase". Carbohydrate Research, 344, 1670-1675.

4- Delattre, C., Pierre, G., Gardarin, C., Traikia, M., Elboutachfaiti, R., Isogai, A., & Michaud, P. (2015). "Antioxidant activities of a polyglucuronic acid sodium salt obtained from TEMPO-mediated oxidation of xanthan". Carbohydrate Polymers, 116, 34-41.

5- Elboutachfaiti, R., Delattre, C., Petit, E., & Michaud, P. (2011). "Polyglucuronic acids: Structures, functions and degrading enzymes". Carbohydrate Polymers, 84, 1-13. 6- Courtois-Sambourg, J., & Courtois, B. (2000). *"Use of glucuronan as an immunostimulating agent and process for its preparation".* FR2781673.

7- Courtois-Sambourg, J., Courtois, B., Heyraud, A., Colin-Morel, P., & Rinaudo-Duhem, M. (1993). *"Polymer compounds of the glycuronic acid, method of preparation and utilization particularly as gelifying, thickenning, hydrating, stabilizing, chelating or floculating means".* WO1993/18174.

8- Courtois, J. and Courtois,B. (1998). *"Use of glucuronan oligo- or polysaccharides, especially produced by Rhizobium meliloti, as cytokine production stimulants for preparing immunostimulant agents".* FR2781673.

9- Lintner, K. (1999). *"Composition for cosmetic* or *dermopharmaceutical use containing a combination of algae extract and exoplysaccharides".* WO9913855.

10- Fournial, A., Grizaud, C.M., LeMoigne, C., & Mondon, P. (2008). *"Cosmetic composition containing acetylated oligoglucuronans".* WO2010/067327.

11- Petit, E., Papy-Garcia, D., Muller, G., Courtois, B., Caruelle, J.P., & Courtois, J. (2004). "Controlled sulfatation of natural anionic bacterial polysaccharides can yield agents with specific regenerating activity in vivo". Biomacromolecules, 5, 445-452.

12- Sakai, S., & Kawakami, K. (2007). "Synthesis and characterization of both ionically and enzymatically cross-linkable alginate". Acta Biomaterialia, 3(4), 495-501.

13- Kurisawa, M., Chung, J. E., Yang, Y. Y., Gao, S. J., & Uyama, H. (2005). "Injectable biodegradable hydrogels composed of hyaluronic acid-tyramine conjugates for drug delivery and tissue engineering". Chemical Communications (Cambridge), 34(34), 4312-4314.

14- Sakai, S., Hirose, K., Taguchi, K., Ogushi, Y., & Kawakami, K. (2009). "An injectable, in situ enzymatically gellable, gelatin derivative for drug delivery and tissue engineering". Biomaterials, 30(20), 3371-3377.

15- Jin, R., Hiemstra, C., Zhong, Z., & Feijen, J. (2007). "Enzyme-mediated fast in situ formation of hydrogels from dextran-tyramine conjugates". Biomaterials, 28(18), 2791-2800.

16- Sakai, S., Tsumura, M., Inoue, M., Koga, Y., Fukano, K., & Taya, M. (2013). "Polyvinyl alcohol -based hydrogel dressing gellable on-wound via a co-enzymatic reaction triggered by glucose in the wound exudate". Journal of Materials Chemistry B, 1(38), 5067-5075.

17- Sakai, S., Mochizuki, K., Qu, Y., Mail, M., Nakahata, M., & Taya, M. (2018). "Peroxidase-catalyzed microextrusion bioprinting of cell-laden hydrogel constructs in vaporized ppm-level hydrogen peroxide". Biofabrication, 10(4), 045007.

18- Sakai, S., Ueda, K., Gantumur, E., Taya, M., & Nakamura, M. (2018). "Drop-On-Drop multimaterial 3D bioprinting realized by peroxidase-mediated cross-linking". Macromolecular Rapid Communications, 39(3), 1700534.

19- Tai, C., Bouissil, S., Gantumur, E., Carranza, M.S., Yoshii, A., Sakai, S., Pierre, G., Michaud, P., & Delattre, C. (2019). "Use of natural polysaccharides in the development of 3D bioprinting technology". Applied Sciences, 9(13), 2596.

20- Gungor-Ozkerim, P. S., Inci, I., Zhang, Y. S., Khademhosseini, A., & Dokmeci, M. R. (2018). "Bioinks for 3D bioprinting: an overview". Biomaterials Science, 6(5), 915-946.

21- Murphy, S. V., & Atala, A. (2014). "3D bioprinting of tissues and organs". Nature Biotechnology, 32(8), 773-785.

## Claims

1. Polymeric compound of glucuronic acid comprising at least one phenolic group.

2. Polymeric compound of glucuronic acid according to claim 1, wherein the said phenolic group is a phenolic hydroxyl group X-(Y-amino-Z-hydroxyalkyl)-n-hydroxyl-phenol of formula (I)

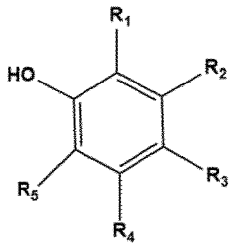

(I)

with:

- $R_1$, $R_2$, $R_4$ and $R_5$ designating a possible hydroxyl group and/or a hydrogen, and/or carboxyl and/or carbonyle and/or alkylphenol and/or aminoalkyl and/or $C_1$ to $C_{20}$ aminohydroxyalkyl and/or $C_1$ to $C_{20}$ aminoalkylphenol
- n designating the number of possible hydroxyl groups and being an integer varying from 0 to 4,
- $R_3$ designating a $C_1$ to $C_{20}$ aminohydroxyalkyl group or a $C_1$ to $C_{20}$ aminoalkyl.

3. Polymeric compound of glucuronic acid according to claim 2, wherein the said phenolic group is selected from the group constituted by tyramine, dopamine and octopamine, and preferably tyramine.

4. Polymeric compound of glucuronic acid according to anyone of claims 2 or 3, wherein said phenolic hydroxyl group of formula (I) is grafted on a α or β-D-glucuronic unit of formula (B)

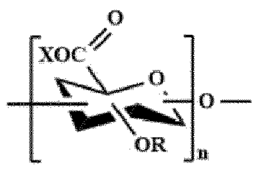

(B)

or its monovalent ion carboxylate salt of formula (A)

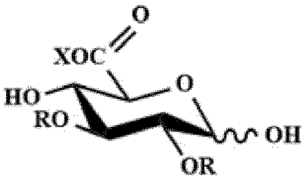

(A)

being bound either:

- (1) in β-(1,4); and/or
- (2) in α-(1,4); and/or
- (3) in β-(1,3); and/or
- (4) in α-(1,3); and/or
- (5) in α-(1,2) and/or
- (6) in β-(1,4)

with:

X= designating amonovalent ion M+ of a metal belonging to the group of alkali metals, and
R= designating H and/or sulfate group and/or an acyl group including $COCH_3$, and
n being an integer chosen so that the molar mass of the polymeric compound is comprised between 10 to 1000 kilodaltons.

**5.** Method for producing a polymeric compound of glucuronic acid according to anyone of claims 1 to 4, said method comprising the steps of:

- providing a radical of a polymeric compound of glucuronic acid of formula (C)

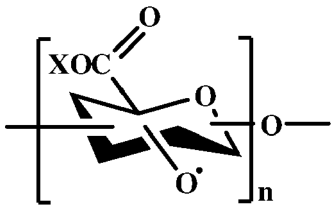

(C);

- reaction of said radical of formula (C) with a phenolic compound of formula (I)or(D)

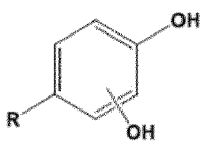

(D)

by radical coupling so as to obtain a polymeric compound of glucuronic acid of formula (E)or(F)

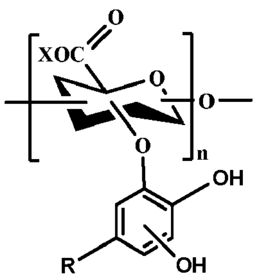

(E)

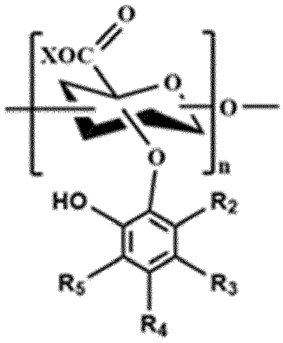

(F)

**6.** Method for producing a polymeric compound of glucuronic acid according to claim 4, said method comprising the steps of:

- providing a polymeric compound of glucuronic acid (PGU) of formula (A) or (B) as defined in claim 5 ;
- dissolving said polymeric compound of glucuronic acid in an acid buffered solution for forming a solution of PGU;
- sequentially adding, in said solution of PGU, a X-(Y-amino-Z-hydroxyalkyl)-n-hydroxyl-phenol or a salt derivative thereof, NHS and WSCD, for forming the final composition of the reaction medium;
- stirring said final composition for at least 4 hours at room temperature until obtaining a resultant polymer consisting in a polymeric compound of glucuronic acid comprising phenolic hydroxyl moieties.

**7.** Method according to anyone of claims 4 and 5, wherein said X-(Y-amino-Z-hydroxyalkyl)-n-hydroxyl-phenol or a salt derivative thereof or said phenolic compound of formula (D) is selected from the group constituted by tyramine, dopamine and octopamine, and preferably tyramine.

**8.** Solution comprising water and a polymeric compound of glucuronic acid as defined in anyone of claims 1 to 4 or obtainable by the method according to anyone of claims 5 to

**9.** Gel-forming composition comprising:

- a polymeric compound of glucuronic acid as defined in anyone of claims 1 to 4 or obtainable by the method according to anyone of claims 5 to 7, and
- a cross-linking catalyst consisting in an oxidase or a compound containing $H_2O_2$ or capable of generating $H_2O_2$ *in situ.*

**10.** Gel-forming composition according to claim 9, wherein said polymeric compound of glucuronic acid is associated to an oxidase as cross-linking catalyst is chosen from the group consisting of oxydo-reductase, peroxidase, catalase, tyrosinase and monosaccharide oxidase, and the mixtures thereof, and is preferably horseradish peroxidase (HRP).

**11.** Gel-forming composition according to claim 10, wherein said oxidase is a horseradish peroxidase (HRP) present in an amount of at least 0.01 U/mL, preferably comprised between 0.1 U/mL and 20 U/mL in the composition, and even better in the order of 5 U/mL.

**12.** Gel-forming composition according to claim 9, wherein said polymeric compound of glucuronic acid is associated to a compound containing $H_2O_2$ in an amount comprised between 0,05 mmol/L and 1 mmol/L.

**13.** Gel-forming composition according to anyone of claims 9 to 12, wherein said polymeric compound of glucuronic acid is present in amount comprised between 0.01 w/v % to 8 w/v %, and preferably 0. 1 w/v % to 2 w/v %.

**14.** Gel-forming composition according to anyone of claims 9 to 13, further comprising another biodegradable polymer.

**15.** Gel-forming composition according to claim 14, wherein said biodegradable polymer is a polysaccharide or a protein selected from the group consisting of collagen, adhesin, gelatin, and the mixtures thereof.

**16.** Gel-forming composition according to claim 15, wherein said biodegradable polymer is a gelatin derivative comprising phenolic hydroxyl moieties (Gelatin-Ph).

**17.** Gel-forming composition according to anyone of claims 9 to 13, further comprising suspended cells of animal, bacterial or plant origin.

**18.** Method for manufacturing an hydrogel structure, comprising

- providing a gel-forming composition as defined in anyone of claims 9 to 16,
- hydrogelating said gel-forming composition:

  ◦ either by contact with a fluid or gaseous medium containing $H_2O_2$ if the gel-forming composition comprises an oxidase;
  ◦ or by contact with an oxidase if the gel-forming composition comprises a compound containing $H_2O_2$ or capable of generating $H_2O_2$ *in situ.*

**19.** Method for manufacturing an hydrogel structure, comprising

- providing a gel-forming composition as defined in claim 17,
- hydrogelating said gel-forming composition:

  ◦ either by contact with a fluid or gaseous medium containing $H_2O_2$ if the gel-forming composition comprises an oxidase;
  ◦ or by contact with an oxidase if the gel-forming composition comprises a compound containing $H_2O_2$ or capable of generating $H_2O_2$ *in situ.*

**20.** Method according to anyone of claims 18 or 19, consisting in a bioprinting process, for manufacturing one dimensional hydrogel structures, or two-dimensional hydrogel structures, or three-dimensional hydrogel structures.

**21.** Hydrogel structure obtainable by the method according to claims 18 and 20 in combination.

**22.** Cell-containing hydrogel structure obtainable by the method according to claims 19 and 20 in combination.

**23.** Use of the hydrogel structure according to claim 21 as three-dimensional cell culture material to support cell colonisation.

**24.** Use of the hydrogel structure according to claim 21 as a patch or a plaster.

**25.** Use of the cell-containing hydrogel structure according to claim 23 for tissue regeneration and/or tissue engineering.

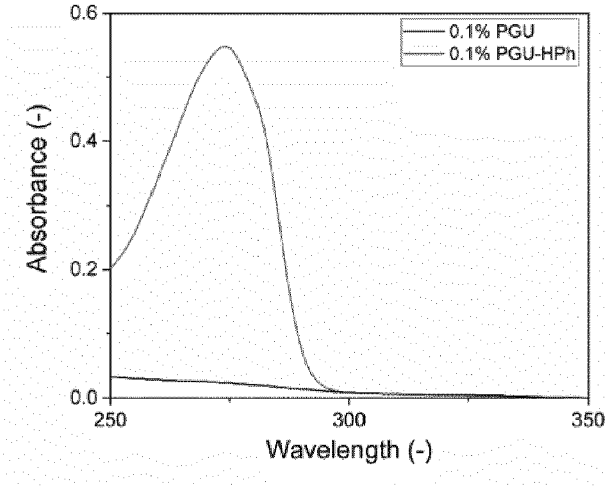


FIG.1

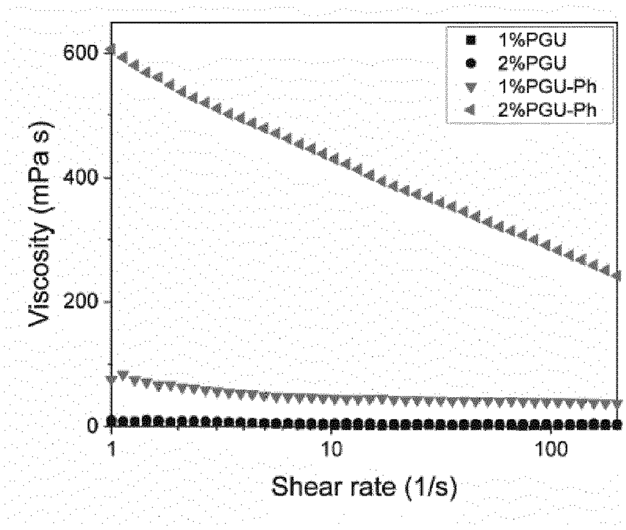


FIG.2

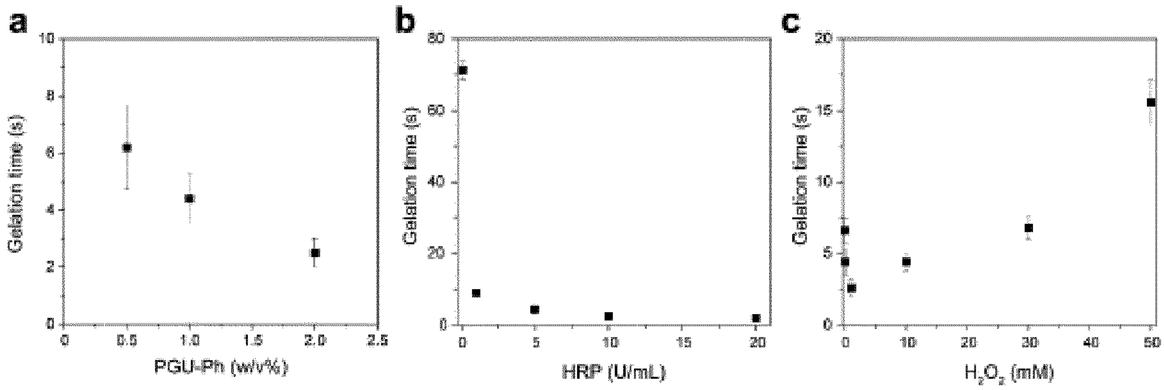


FIG.3

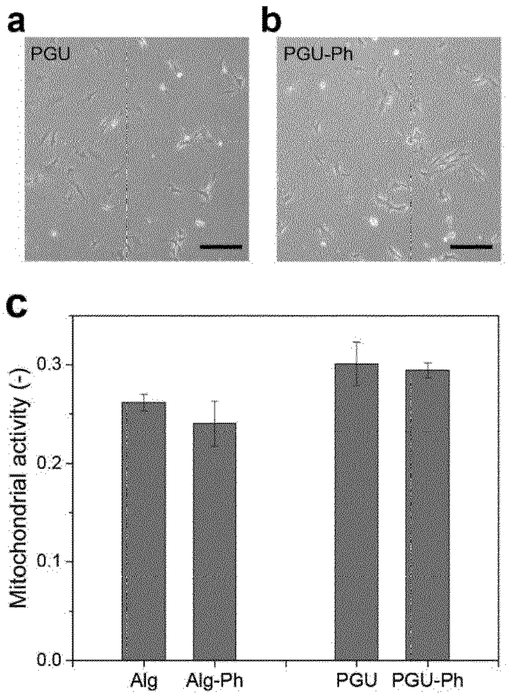


FIG. 4

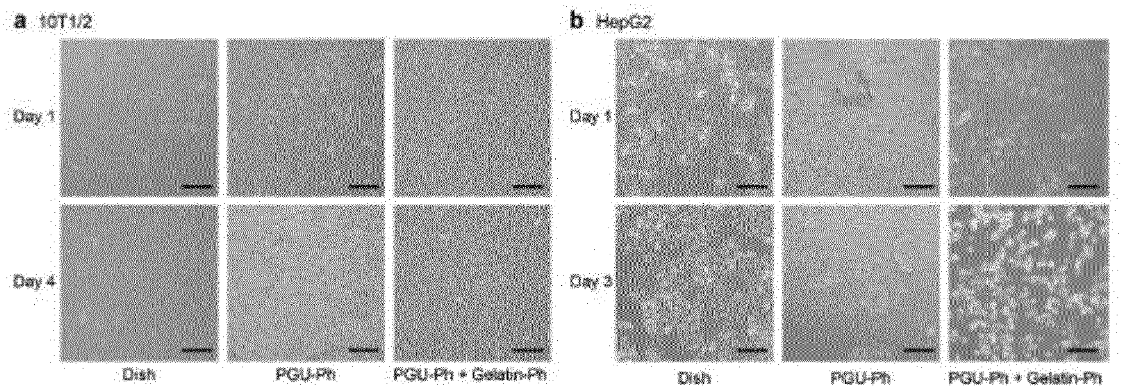


FIG.5

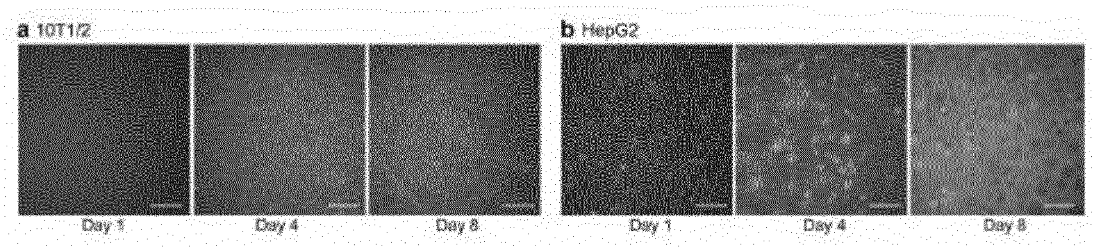


FIG.6

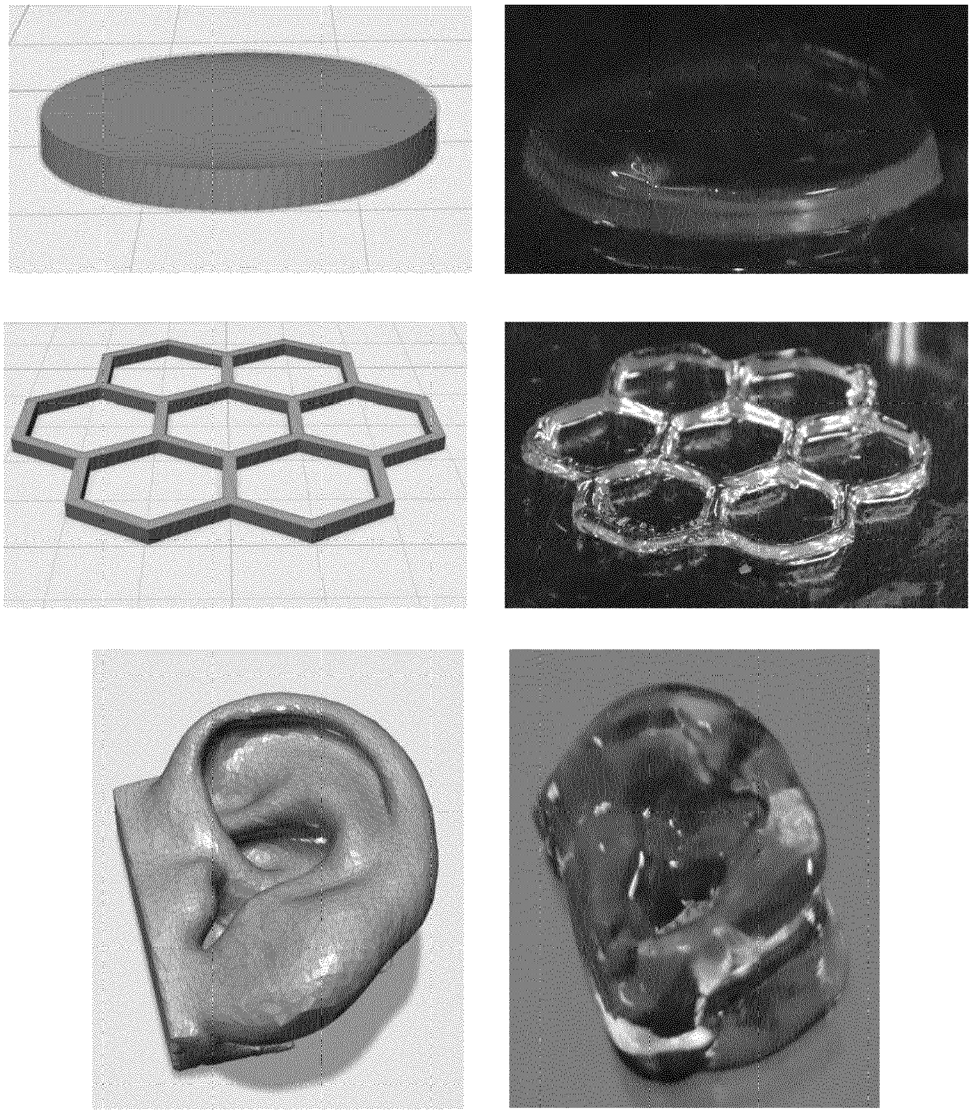

FIG.7

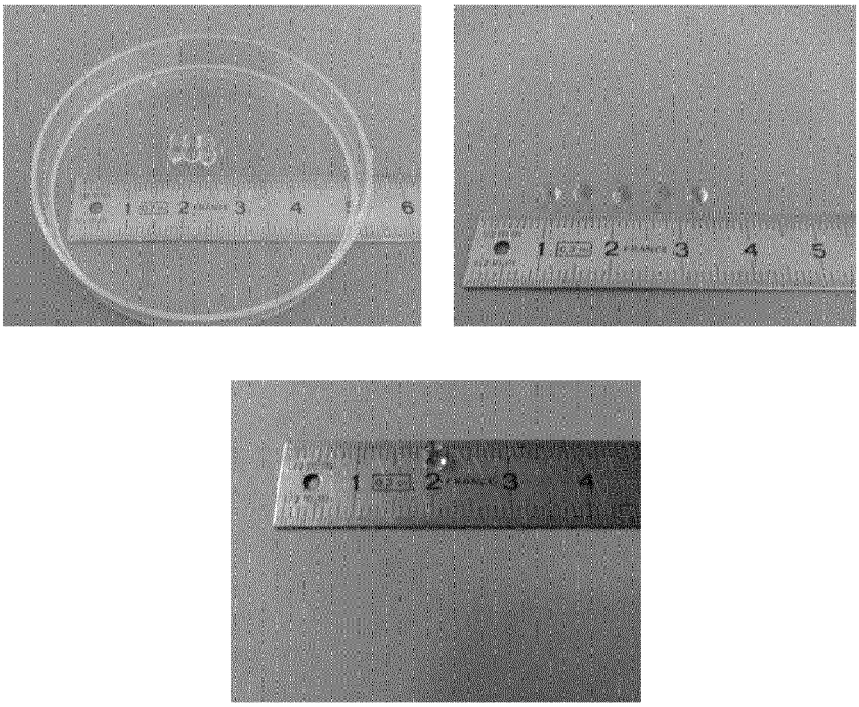

FIG.8

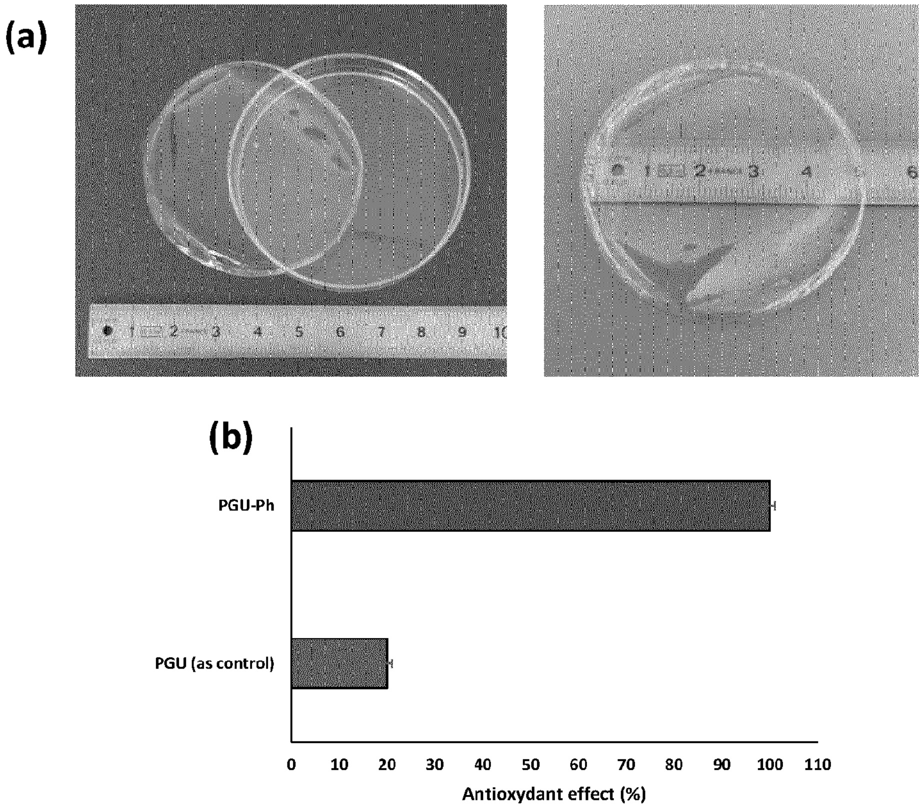
(a)
(b)
PGU-Ph
PGU (as control)
0 10 20 30 40 50 60 70 80 90 100 110
Antioxydant effect (%)

FIG.9

Europäisches Patentamt
European Patent Office
Office européen des brevets

# EUROPEAN SEARCH REPORT

Application Number
EP 21 30 5612

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZIADLOU REIHANE ET AL: "Optimization of hyaluronic acid-tyramine/silk-fibroin composite hydrogels for cartilage tissue engineering and delivery of anti-inflammatory and anabolic drugs", MATERIALS SCIENCE AND ENGINEERING C, ELSEVIER SCIENCE S.A, CH, vol. 120, 5 November 2020 (2020-11-05), XP086478793, ISSN: 0928-4931, DOI: 10.1016/J.MSEC.2020.111701 [retrieved on 2020-11-05] | 1-4,8-25 | INV. C12N5/00 A61L27/52 C08L5/00 C08B37/00 C08B37/08 C08L5/08 A61K47/36 |
| A | * abstract; fig.1A; paragraphs 2.1, 2.3 and 2.5 * | 5-7 | |
| | ----- | | |
| X | WO 2004/063388 A2 (CLEVELAND CLINIC FOUNDATION [US]; CALABRO ANTHONY [US] ET AL.) 29 July 2004 (2004-07-29) | 1-4,8-25 | |
| A | * abstact; page 4, para.3; scheme on page 13; page 18, para.2-page 19, para.1; claims 1, 10, 16, 18, 21, 55 * | 5-7 | |
| | ----- | | |

| TECHNICAL FIELDS SEARCHED (IPC) |
|---|
| C12N |
| A61L |
| C08L |
| C08B |
| A61K |

The present search report has been drawn up for all claims

1

| Place of search | Date of completion of the search | Examiner |
|---|---|---|
| Munich | 1 October 2021 | Pellegrini, Paolo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 21 30 5612

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2021

| Patent document cited in search report | Publication date | Patent family member(s) | Publication date |
|---|---|---|---|
| WO 2004063388 A2 | 29-07-2004 | AU 2004204465 A1 | 29-07-2004 |
| | | CA 2512730 A1 | 29-07-2004 |
| | | CN 1826381 A | 30-08-2006 |
| | | CN 101366974 A | 18-02-2009 |
| | | EP 1587945 A2 | 26-10-2005 |
| | | JP 5325385 B2 | 23-10-2013 |
| | | JP 2006517598 A | 27-07-2006 |
| | | US 2004147673 A1 | 29-07-2004 |
| | | US 2005265959 A1 | 01-12-2005 |
| | | WO 2004063388 A2 | 29-07-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2781673
- WO 199318174 A
- WO 2010067327 A
- WO 9913855 A


### Non-patent literature cited in the description


- **HEYRAUD, A. ; COURTOIS, J. ; DANTAS, L. ; COLIN-MOREL, P. ; COURTOIS, B.** Structural characterization and rheological properties of an extracellular glucuronan produced by a Rhizobium meliloti M5N1 mutant strain. *Carbohydrate Research,* 1993, vol. 240 (24), 71-78
- **DE RUITER, G.A. ; JOSSO, S.L. ; COLQUHOUN, I.J. ; VORAGEN, A.G.J. ; ROMBOUTS, F.M.** Isolation and characterization of β-(1-4)-D-glucuronans from extracellular polysaccharides of moulds belonging to Mucorales. *Carbohydrate Polymers,* 1992, vol. 18 (1), 1-7
- **ELBOUTACHFAITI, R. ; DELATTRE, C. ; PETIT, E. ; EL GADDA, M. ; COURTOIS, B. ; MICHAUD, P. ; EL MODAFAR, C. ; COURTOIS, J.** Improved isolation of glucuronan from algae and the production of glucuronic acid oligosaccharides using a glucuronan lyase. *Carbohydrate Research,* 2009, vol. 344, 1670-1675
- **DELATTRE, C. ; PIERRE, G. ; GARDARIN, C. ; TRAIKIA, M. ; ELBOUTACHFAITI, R. ; ISOGAI, A. ; MICHAUD, P.** Antioxidant activities of a polyglucuronic acid sodium salt obtained from TEMPO-mediated oxidation of xanthan. *Carbohydrate Polymers,* 2015, vol. 116, 34-41
- **ELBOUTACHFAITI, R. ; DELATTRE, C. ; PETIT, E. ; MICHAUD, P.** Polyglucuronic acids: Structures, functions and degrading enzymes. *Carbohydrate Polymers,* 2011, vol. 84, 1-13
- **PETIT, E. ; PAPY-GARCIA, D. ; MULLER, G. ; COURTOIS, B. ; CARUELLE, J.P. ; COURTOIS, J.** Controlled sulfatation of natural anionic bacterial polysaccharides can yield agents with specific regenerating activity in vivo. *Biomacromolecules,* 2004, vol. 5, 445-452
- **SAKAI, S. ; KAWAKAMI, K.** Synthesis and characterization of both ionically and enzymatically cross-linkable alginate. *Acta Biomaterialia,* 2007, vol. 3 (4), 495-501
- **KURISAWA, M. ; CHUNG, J. E. ; YANG, Y. Y. ; GAO, S. J. ; UYAMA, H.** Injectable biodegradable hydrogels composed of hyaluronic acid-tyramine conjugates for drug delivery and tissue engineering. *Chemical Communications,* 2005, vol. 34 (34), 4312-4314
- **SAKAI, S. ; HIROSE, K. ; TAGUCHI, K. ; OGUSHI, Y. ; KAWAKAMI, K.** An injectable, in situ enzymatically gellable, gelatin derivative for drug delivery and tissue engineering. *Biomaterials,* 2009, vol. 30 (20), 3371-3377
- **JIN, R. ; HIEMSTRA, C. ; ZHONG, Z. ; FEIJEN, J.** Enzyme-mediated fast in situ formation of hydrogels from dextran-tyramine conjugates. *Biomaterials,* 2007, vol. 28 (18), 2791-2800
- **SAKAI, S. ; TSUMURA, M. ; INOUE, M. ; KOGA, Y. ; FUKANO, K. ; TAYA, M.** ''Polyvinyl alcohol -based hydrogel dressing gellable on-wound via a co-enzymatic reaction triggered by glucose in the wound exudate. *Journal of Materials Chemistry B,* 2013, vol. 1 (38), 5067-5075
- **SAKAI, S. ; MOCHIZUKI, K. ; QU, Y. ; MAIL, M. ; NAKAHATA, M. ; TAYA, M.** Peroxidase-catalyzed microextrusion bioprinting of cell-laden hydrogel constructs in vaporized ppm-level hydrogen peroxide. *Biofabrication,* 2018, vol. 10 (4), 045007
- **SAKAI, S. ; UEDA, K. ; GANTUMUR, E. ; TAYA, M. ; NAKAMURA, M.** Drop-On-Drop multimaterial 3D bioprinting realized by peroxidase-mediated cross-linking. *Macromolecular Rapid Communications,* 2018, vol. 39 (3), 1700534
- **TAI, C. ; BOUISSIL, S. ; GANTUMUR, E. ; CARRANZA, M.S. ; YOSHII, A. ; SAKAI, S. ; PIERRE, G. ; MICHAUD, P. ; DELATTRE, C.** Use of natural polysaccharides in the development of 3D bioprinting technology. *Applied Sciences,* 2019, vol. 9 (13), 2596
- **GUNGOR-OZKERIM, P. S. ; INCI, I. ; ZHANG, Y. S. ; KHADEMHOSSEINI, A. ; DOKMECI, M. R.** Bioinks for 3D bioprinting: an overview. *Biomaterials Science,* 2018, vol. 6 (5), 915-946

- **MURPHY, S. V. ; ATALA, A.** 3D bioprinting of tissues and organs. *Nature Biotechnology,* 2014, vol. 32 (8), 773-785